## Europäisches Patentamt
## European Patent Office
## Office européen des brevets

(19)

(11) Publication number: **0 242 007**
**B1**

(12)

# EUROPEAN PATENT SPECIFICATION

(45) Date of publication of the patent specification:
07.11.90

(51) Int. Cl.⁵: **C12N 9/04**, C12N 15/00,
C11D 3/386

(21) Application number: 87201055.8

(22) Date of filing: 05.06.87

(54) Process for preparing a catalase-free oxidase and a catalase-free oxidase-containing yeast, and use thereof.

(30) Priority: 24.11.86 NL 8602978

(43) Date of publication of application:
21.10.87 Bulletin 87/43

(45) Publication of the grant of the patent:
07.11.90 Bulletin 90/45

(84) Designated Contracting States:
AT BE CH DE ES FR GB GR IT LI NL SE

(56) References cited:
EP-A- 0 019 937
EP-A- 0 071 990
EP-A- 0 173 378
FR-A- 2 218 346
US-E- 32 016

CHEMICAL ABSTRACTS, vol. 93, no. 21, 24th
November 1980, page 348, abstract no. 200741z,
Columbus, Ohio, US; L. EGGELING et al.: "Regulation
of alcohol oxidase synthesis in Hansenula polymorpha:
oversynthesis during growth on mixed substrates and
induction by methanol", & ARCH.
MICROBIOL. 1980, 127(2), 119-24 000
CHEMICAL ABSTRACTS, vol. 96, no. 9, 1st March 182,
page 301, abstract no. 65423s, Columbus, Ohio, US; L.
EGGELING et al.: "Enhanced utilization-rate of

(73) Proprietor: UNILEVER NV, Burgemeester
s'Jacobplein 1 P.O. Box 760, NL-3000 DK Rotterdam(NL)
(84) Designated Contracting States: BE CH DE ES FR GR IT LI
NL SE AT

(73) Proprietor: UNILEVER PLC, Unilever House Blackfriars
P.O. Box 68, London EC4P 4BQ(GB)
(84) Designated Contracting States: GB

(72) Inventor: Giuseppin, Marco Luigi Federico,
Munterstraat 13, NL-3123 PN Schiedam(NL)

(74) Representative: van der Toorren, Johannes, Drs. et al,
UNILEVER N.V. Patent Division Postbus 137,
NL-3130 AC Vlaardingen(NL)

(56) References cited: (continuation)
methanol during growth on a mixed substrate: a
continuous culture study with Hasenula polymorpha",
& ARCH.
MICROBIOL. 1981, 130(5), 362-5 1980, 127(2), 119-24 000
CHEMICAL ABSTRACTS, vol. 88, no. 25, 19th June 1978,
page 287, abstract no. 185105w, Columbus, Ohio, US;
G.T. MIWA et al.: "The direct oxidation of ethanol by a
catalase- and alcohol dehydrogenase-free
reconstituted system containing cytochrome P-450", &
ARCH. BIOCHEM.
BIOPHYS. 1978, 187(2), 464-75(2), 119-24 000
MICROBIOLOGY ABSTRACTS: Section A. Industrial &
Applied Microbiology, vol. 17, no. 4, 1982,
abstract 2547-A17, Oxford, GB; R.N. PATEL et al.:
"Microbial oxidation of methanol: properties of
crystallized alcohol oxidase from a yeast, Pichia sp.", &
ARCH. BIOCHEM. BIOPHYS., vol. 210, no. 2, Sep. 1981,
pp. 481-488 000
CHEMICAL ABSTRACTS, vol. 87, no. 25, 19th
December 1977, page 537, abstract no. 199125u,
Columbus, Ohio, US; V.S. PODGORSKII et al.:
"Production of protein-vitamin concentrates from
methyl alcohol", & REF. NAUCHNO-ISSLED. RAB. INST.
MIKROBIOL. VIRUSOL. AKAD. NAUK UKR.
SSR 1974 (PUB. 1976), 17-19981, pp. 481-488 000

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

**Description**

The invention relates to a process for the preparation of an oxidase by aerobic fermentation of a yeast under conditions in which the yeast produces oxidase, and, if desired, isolation from the yeast cells of the oxidase produced.

Oxidases are enzymes which catalyse specific oxidation reactions of certain substrates and thereby produce $H_2O_2$. An example of an oxidase is methanol oxidase (MOX) that enables methylotrophic yeasts such as Hansenula polymorpha, Candida boidinii, Pichia pastoris and suchlike to grow on methanol. The MOX catalyses the oxidation of methanol to formaldehyde and hydrogen peroxide, during which molecular oxygen acts as electron acceptor. The formaldehyde formed is used for dissimilation (catabolism), during which it is converted into formic acid and finally into carbon dioxide, or used for assimilation (anabolism), during which cell material is produced via the xylulose monophosphate route. With these further processes other enzymes play an important rôle: in the assimilation process dihydroxyacetone synthetase is important, in the dissimilation process formaldehyde dehydrogenase and formiate dehydrogenase are key enzymes. The hydrogen peroxide formed by the oxidase is extremely toxic for these organisms and, according to the present state of art, is immediately rendered innocuous by another enzyme, namely catalase.

Other examples of oxidases are other alcohol oxidases, glucose oxidase, glycerol oxidase, D-aminoacid oxidase, aldehyde oxidase, amine oxidase, arylalcohol oxidase, galactose oxidase, sorbose oxidase, uric acid oxidase and xanthine oxidase.

Oxidases can be used for various purposes. An important use lies in the field of washing and bleaching compositions, where the presence of oxidase during the washing or bleaching process can lead to the in situ formation of hydrogen peroxide if at the same time a suitable substrate for the oxidase is present. The hydrogen peroxide displays a bleaching agent activity, or can, in co-operation with bleaching agent precursors such as TAED (tetraacetylethylenediamine), lead to the formation of bleaching agents which are active at low temperatures, such as peracids. An important advantage of this system, in which the washing or bleaching agent itself contains no hydrogen peroxide but only the enzyme that during the washing or bleaching process catalyses the formation of hydrogen peroxide in situ, is that inactivation of washing composition components, such as proteases, lipases and suchlike, during storage and transport is prevented. This use of oxidases is described in the British patent publications 1 225 713 and 2 101 167 and in the German patent publication 2 557 623.

Another use of oxidases lies in the field of qualitative and/or quantitative analysis, e.g. for determining the alcohol content of systems which are to be investigated, such as fermentation liquids. The analysis is based on a measurement of the hydrogen peroxide formed via an oxidation of colouring substances catalysed by peroxidase.

Oxidases can also be used within the framework of chemical syntheses or of processes for the purification of refuse in order to catalyse oxidation of substrates.

For the most of these uses, and certainly for that as component of washing or bleaching compositions and for that as aid in analysis, it is of great importance that the oxidase is free of catalase, which would decompose the hydrogen peroxide formed. A well-known problem with the microbiologically produced oxidases such as methanol oxidase (EC 1.1.3.13) by fermentation of methylotrophic yeasts such as Hansenula polymorpha, Candida boidinii, Pichia pastoris and suchlike on methanol, is, however, that they are constantly accompanied by the natural catalase enzyme [Veenhuis, M., van Dijken, J.P. and Harder, W. (1983) in Advances in Microbial Physiology, Rose, A.H., Gareth Morris, J. and Tempest, D.W., Editors, Vol. 24, pp 1-82, Academic Press, New York] that almost immediately decomposes the peroxide formed, so that no effective bleaching agent activity or accurate analysis of the substrate concentration is possible. It is true that methods are known (see for example the British patent specification 2 101 167) for removing the catalase from the product to a great extent or for inactivating it and thus obtaining an active substantially catalase-free oxidase, but for technical application these methods (such as catalase removal through ion exchange chromatography or gel filtration, and catalase inactivation through treatment with sodium dodecylsulphate (SDS) or sodium azide, and so on) are far too expensive and cumbersome and in many cases inadequate, or, in the case of sodium azide, a toxic material is even introduced.

Consequently there is need of a method with which catalase-free oxidase can be obtained in a technically and economically justifiable manner. A microbiological production, in which catalase-negative mutants are used, could meet this need, but seems impracticable because, on the basis of the literature, it could be expected that the organisms without catalase are doomed to die if cultured in a nutritive medium to which substrate for the oxidase has been added in behalf of a good growth of the microorganism and strong expression of the oxidase gene.

In the case of methanol oxidase (MOX) it is known that the formation of MOX, formiate dehydrogenase, formaldehyde dehydrogenase, dihydroxyacetone synthetase and catalase is subject to glucose repression, as well as to glycerol and mannitol repression. Derepression of MOX synthesis occurs if the organisms are cultured at very low glucose, glycerol or mannitol concentrations, but the MOX production is then still relatively very small.

For a good understanding of the previous and the following paragraphs some definitions are appropriate.

An inductor is a compound that promotes the transcription of a gene by an interaction with the promoter, by inactivation of the effective repressor or by blocking of a repressor gene.

A repressor is a compound that blocks the transcription of a gene by an interaction with the promoter or by an activation of a repressor protein gene.

Synthesis of a gene product (transcription of a gene) can occur by effects of an inducer (induction) or by relieving the repressor-mediated repression (derepression).

In the case of MOX, methanol acts as an inducer in both batch and continuous cultures. Glycerol, sorbitol, glucose and ethanol are known repressors in batch and continuous cultures.

Under conditions with low concentrations of glycerol, sorbitol and glucose in batch cultures and in steady state continuous cultures at low dilution rates a derepression of MOX occurs.

It is known that complete induction of the MOX synthesis only takes place when the organisms are cultured on methanol as only source of carbon or on specific mixtures of methanol with glucose, glycerol or mannitol [see Eggeling and Sahm, Arch. Microbiol. 127 (1980) 119-124 and Arch. Microbiol. 130 (1981) 362-365]. These authors conclude from their experiments on the yeast Hansenula polymorpha that methanol itself, and not a metabolite thereof, is responsible for induction of MOX synthesis. From these publications it can be gathered that the level of expression in the situation of repression or derepression is low compared with the level of expression in the situation of induction: in the order of magnitude of 1-2% under complete glucose repression, and about 10% under glucose or glycerol derepression at low growth rates in continuous cultures. Indeed, in one of these publications [Arch. Microbiol. 127 (1980) 119-124], a catalase-negative mutant of Hansenula polymorpha is described, indicated as mutant 55/11, which mutant cannot grow on methanol, but produces methanol oxidase when grown on glycerol under conditions of derepression. In batch cultures the MOX production of the catalase negative mutant was about 50% of the amount which is produced on glycerol by the wild-type, i.e. also catalase-producing, strain. Compared with the MOX production of the wild-type strain on methanol, the MOX production of the catalase-negative mutant was only about 17%. Such a relatively small productivity of the preparation of catalase-free oxidase is inadequate for production on a technical scale.

In addition to the literature mentioned above, some other less relevant publications are worth being mentioned. In Chemical Abstracts 88 (1978) 185105w, referring to G.T. Miwa et al., Arch. Biochem. Biophys. 187 (1978) 464-475, the direct oxidation of ethanol by a catalase- and alcohol dehydrogenase-free reconstituted system containing cytochrome P-450 and NADPH is described. In view of the required presence of NADPH, this system will not work as an oxidase like MOX capable of oxidizing alcoholic compounds with molecular oxygen, so that a different system is described which does not give any information on or direction as to the use of a catalase-negative mutant of a yeast producing an oxidase.

In EP-A- 0 019 937 (PHILLIPS PETROLEUM CO.) a method of producing alcohol oxidase is described, wherein a methanol-utilizing Pichia-type micro-organism, e.g. Pichia pastoris, is used. Also enzyme preparations from a methanol-utilizing micro-organism of genus Pichia, catalyzing the reaction

$$RCH_2OH + O_2 \rightarrow RCHO + H_2O_2$$

wherein $R = H$, $CH_3$, $C_2H_5$ or $C_3H_7$, are described, whereby a catalase-free enzyme is produced after several purification treatments. This method suffers from the disadvantage mentioned earlier in this specification. Moreover, the patent publication itself suggests that some catalase is still present in the purified alcohol oxidase.

In EP-A- 0 071 990 (PHILLIPS PETROLEUM CO.) the removal of ambient oxygen from aqueous liquids is described, which removal is catalyzed by enzymatic deoxygenating systems comprising alcohol oxidase in the presence of alcohol optionally with catalase. Although yeasts of the genus Hansenula and the genus Pichia can be used, the use of a catalase-negative mutant according to the present invention has neither been described nor suggested. On the contrary, the presence of catalase is allowed, thus the nature and the purpose of the enzyme preparations used are different from those according to the present invention.

Now it has been found, surprisingly, that a microbiological production of methanol oxidase that is free of catalase can be realized on a technical scale with a process of the kind mentioned in the preamble, when a catalase-negative mutant of the yeast Hansenula polymorpha is allowed to grow in a nutritive medium suitable for the yeast in the presence of methanol and another source of carbon such as glucose, in which methanol induces the expression of the oxidase gene and can also be substrate for the oxidase beside the other source of carbon, while the toxic effects of the peroxide formed are prevented by using a suitable mixing ratio of methanol to other source of carbon. Although it is not clear what has happened exactly during the formation of the catalase-negative mutant, it could quite well be possible that several mutations have occurred simultaneously. For instance, after closer examination it has turned out that the catalase-negative mutant of Hansenula polymorpha has an increased content of cytochrome C-peroxidase that can perhaps render the formed $H_2O_2$ innocuous in a manner similar to that in which catalase does this. It is not yet known whether this is caused by a mutation or by an adaptation of the catalase-negative yeast.

Instead of the catalase-negative Hansenula polymorpha indicated above, other catalase-negative yeasts can also be used. Depending on the oxidase to be produced, a compound other than methanol should also be used as induction agent, that can also be substrate, for example methyl amine in the preparation of amine oxidase. Consequently, the invention is not limited to the production of methanol oxidase.

Various sources of carbon can be used instead of glucose. Therefore, in a general sense the invention embraces a process for the preparation of an oxidase- containing composition by aerobic fermentation of a yeast under conditions in which the yeast produces oxidase, and, if desired, isolation from the yeast cells of the oxidase produced, which is characterized in that a catalase-negative mutant of a yeast is allowed to grow in a nutritive medium suitable for the yeast in the presence of:

a) a so-called inducing substrate that induces expression of the oxidase gene and may also be substrate for the oxidase, and

b) another source of carbon that is suitable for the yeast species chosen,

the molar ratio of inducing substrate to other source of carbon being such that the yeast formed and the oxidase formed do not suffer any harmful effects from the oxidation of the inducing substrate.

In this description, "composition" means both a simple mixture of various components and a preparation in which the oxidase is packed, as it were, for example in encapsulated form.

It is preferred according to the invention that a catalase-negative mutant of a yeast of the genus Hansenula or of the genus Pichia, in particular of the species Hansenula polymorpha or of the species Pichiapastoris be used, such as the catalase-negative mutant Hansenula polymorpha 55/11, ATCC 46059. The invention is certainly not limited to the use of this one mutant. Other catalase-negative mutants can be obtained by the introduction of any mutations at random and the subsequent selection of the mutants obtained, or by directed mutation methods, such as described inter alia in the European patent application 0 173 378 (A2).

It is further preferred according to the invention that a yeast be used in which the genetic information for the oxidase stands under control of a strong promoter, as a result of which a large yield of oxidase can be realized. An example hereof is the MOX promoter, in particular the MOX promoter of Hansenula polymorpha CBS 4732. This occurs naturally in the Hansenulapolymorpha 55/11, ATCC 46059 yeast described hereinbefore, but with other yeasts this can be achieved by means of known recombinant DNA techniques. The strength of the promoter is apparent from the fact that the MOX content of the wild-type strain of Hansenula polymorpha cultured on methanol is in the order of magnitude of 30% of the cellular protein.

Examples of other strong promoters are the dihydroxyacetone synthetase (DAS) promoter of methylotrophic yeasts and the amine oxidase promoter of yeasts producing amine oxidase.

In connection with production on technical scale, it is preferred according to the invention that the yeast is cultured in a continuous fermentation. However, "batch" fermentation or "fed batch" fermentation is not excluded. By "fed batch" is meant a "batch" fermentation in which the substrate is not added in one go at the beginning, but gradually so as to achieve a controllable substrate concentration during the whole fermentation.

It is preferred, according to the invention, that, as oxidase gene, preferably a methanol oxidase gene is used, for example a methanol oxidase gene that codes for a methanol oxidase with the same amino acid sequence as the known MOX of Hansenula polymorpha CBS 4732, or a derivative of this sequence, obtained via enzyme engineering, or a modification thereof which has no really lesser functionality. In combination with a methanol oxidase, preferably methanol is used as inducing substrate. As well as for methanol, the MOX enzyme also appears to be active for various other substrates, such as for ethanol, n-propanol, n-butanol, n-amylalcohol, and even, though to a slighter extent, for substances such as methylcellosolve, ethylene glycol, benzyl alcohol, isopropanol, isoamyl alcohol and propylene glycol. Some of these substances, such as ethanol, are pre-eminently suitable for serving as substrate when using methanol oxidase in washing and bleaching compositions, because they are relatively cheap and toxicologically acceptable.

During the fermentation, a nutritive medium suitable for the yeast, such as Hansenula or Pichia, is used which must contain a carbon source suitable for this organism. Suitable sources of carbon are known to the expert or can easily be determined by experiment. Glucose, for example, is suitable, but also other sugars, such as sorbose, xylose, sorbitol, and other substances, such as glycerol and the like, can be used as carbon source, while also commercially available sources of carbon such as molasses come into consideration. A suitable nutritive medium is known from Egli et al., Arch. Microbiol. 124 (1980) 115-121.

According to the invention it is important that, during the fermentation, a suitable ratio between the amount of carbon source and the amount of inducing substrate, for example methanol, is established.

A preferred embodiment of the invention is characterized in that the yeast is cultured in the presence of a carbon source suitable for the yeast species chosen, for example glucose, and methanol, the molar ratio of methanol : glucose being (0.025-3):1 and preferably (0.8-1.8):1.

In experiments with such methanol/glucose mixtures in continuous fermentations with a dilution rate D of 0.1 hour$^{-1}$ of the catalase-negative mutant Hansenulapolymorpha 55/11, ATCC 46059, MOX yields have been realized which are in the order of magnitude of 50% of the MOX yield of the catalase-producing wild-type strain cultured on methanol. These are yields which, also in view of the absence of catalase, are suitable for use on technical scale.

According to a preferred embodiment of the invention, the catalase-negative oxidase-containing yeast is, after finishing the continuous fermentation, subjected to a drying treatment, whereby the oxidase enzyme is not inactivated, or another treatment whereby the cells are made permeable or are inactivated. A freeze-drying treatment appeared to be very suitable.

The advantage of such drying treatment is that the cells inactivated by the drying treatment immediately start $H_2O_2$ production when used with methanol concentrations of 5 to at least 300 mmol/l. On the contrary, it was found that a living cell suspension showed a lag time when used with methanol concentrations of 75-300 mmol/l, and below 75 mmol/l no $H_2O_2$ production occurred. This might be caused by the presence of cytochrome C peroxidase in living cell suspensions having a similar $H_2O_2$-decomposing action as catalase, which presence was mentioned before in this specification.

When the concentrations of methanol used are too high and/or the ratio of methanol with respect to glucose is too high, a lower MOX yield is obtained and the culture can even be killed. Concentrations of methanol which are too low give insufficient induction, so that a low yield of oxidase is the result. With growth of the mutant on glucose only, without methanol, the same low MOX activities are obtained as in the case of the wild-type strain, apparently as a result of the glucose repression.

If, for the intended use, one is unwilling or unable to use the cultured yeast cells as such, the oxidase accumulated in the yeast cells and particularly in the peroxisomes should be isolated from the cells. To do this, usual methods of cell lysis can be applied, such as the physical breaking of the cells with the aid of glass globules, treatment in a so-called French press or Manton Gaulin homogenizer, ultrasonic treatment, and enzymatic methods, e.g. with zymolyase. The invention also relates to an oxidase-containing, catalase-negative mutant of a yeast, obtained by making use of a process according to the invention for preparing such a yeast, as well as to an oxidase or an oxidase-containing composition, obtained by isolation of the oxidase from a yeast according to the invention, possibly followed by processing the oxidase thus isolated to a composition with other components.

Further, the invention relates to the use of an oxidase-containing, catalase-negative mutant of a yeast according to the invention or the use of an oxidase isolated therefrom together with a substrate for the oxidase for the in situ production of hydrogen peroxide in a washing or bleaching process. Particularly when an alcohol oxidase is used, ethanol is preferably used as substrate in such a washing process.

Also a washing or bleaching agent that contains an oxidase-containing, catalase-negative mutant of a yeast according to the invention, or the oxidase isolated therefrom, is an embodiment of the invention.

Further, the invention relates to the use of an oxidase-containing, catalase-negative mutant of a yeast according to the invention or of an oxidase isolated therefrom as catalyst for the oxidation of a substrate for the oxidase within the framework of a chemical synthesis or of a process for purifying refuse or for the qualitative and/or quantitative determination of a substrate for the oxidase.

The invention is further explained in the following experimental part.

## Fermentation conditions and media

Hansenula polymorpha was used as a catalase-negative colony from yeast peptone dextrose agar and pre-cultured for one day at 37°C in a medium according to Egli et al., Arch. Microbiol. 124 (1980), 115-121, with glucose as sole carbon source.

Culturing was carried out continuously in a 3-litre fermenter containing 2 litres of medium. The aeration, pH and agitation were regulated, care being taken that the pressure of dissolved oxygen never came below 25% saturation. The pH and foam formation were controlled with the aid of a concentrated ammonia solution which contained an anti-foaming agent based on silicone oil (Rhodorsil 426® R ex Rhone Poulenc) in a ratio of 4:1 to 1:1. The pH was adjusted to 5.0 ± 0.05 pH units. The temperature was kept at 37 ± 0.2°C. The streams of medium entering and leaving were measured and adjusted to a desired flow rate with the aid of a peristaltic pump. The methanol concentration was gradually increased in order to adapt the culture to methanol. Stable states of the fermentation were tested by measuring the respiration parameters (the respiration quotient, the rate of carbon dioxide development and the rate of oxygen absorption), and by testing the MOX contents in cell suspensions and cell lysates.

## Analyses

## Determination of MOX and catalase

The MOX activity in cell suspensions, cell-free extracts and the catalase activity were determined according to van Dijken et al., Arch. Microbiol. 111 (1976), 137-144. 1 unit corresponds with 1 micromol methanol used per minute at 37°C in an air-saturated 0.1 M phosphate buffer having a pH of 7.5.

Protein

Protein was determined according to the method of Lowry et al., J. Biol. Chem. 193 (1951), 265-275. Bovine serum albumin was used as standard.

Biomass

The dry weight of the biomass was determined by drying washed cell suspensions at 100°C to constant weight.

Glucose, methanol, formic acid and formaldehyde were determined with the aid of HPLC and standard enzymatic analyses.

Breaking of cells

Cell lysates were obtained by ultrasonic treatment or by incubation with zymolyase.

Ultrasonic treatment

Ultrasonic treatment was carried out at 0°C with the aid of a 0.1 M potassium phosphate solution having a pH of 7.5, which contained 5 ml of washed cell suspension, with 3 g glass beads (average diameter 100μm). The cell suspension was treated 5 times for 1 minute with a Branson cell breaker type B-12 (Branson Sonic Power Company). Between the treatments, a cooling period of 1 minute was observed. A power of 70 Watt per 5 ml of solution was introduced, for doing which a microtip was used.

Example I - Optimization of the Production of MOX on methanol/glucose mixtures

Hansenula polymorpha ATCC 46059 was grown on the medium as described in Table A, which medium contained various ratios of methanol to glucose.

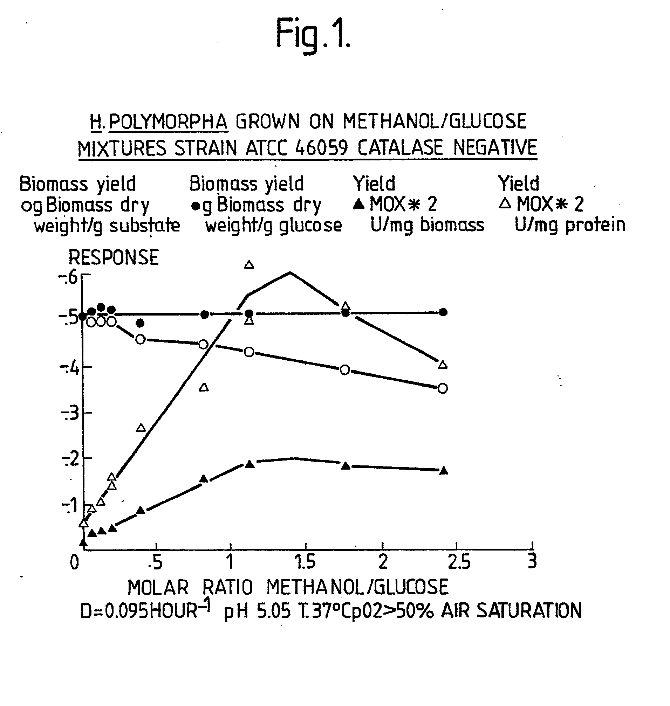

Figure 1 and Table B show the specific MOX activity in the cell lysates, obtained from cells in an equilibrium situation after 4 to 5 periods of residence. At a constant dilution rate of 0.095 hour$^{-1}$, an optimal range of 1-1.8 mol methanol to 1 mol glucose was found from Figure 1 for the ratio of methanol : glucose.

In Table C, values of residual concentrations of methanol, formaldehyde and glucose are indicated. Apparently, with ratios of methanol : glucose above the optimal ratio, the concentrations of methanol and/or formaldehyde become so high that they start acting toxically on the yeast and the oxidase, which results in lower MOX yields, as can be seen in Figure 1.

In the cell lysates obtained from the above-mentioned cultures, no catalase activity could be detected.

The wild-type strain Hansenula polymorpha CBS 4732, from which the catalase-negative mutant Hansenulapolymorpha 55/11, ATCC 46059, has been derived, was cultured under identical conditions on a number of methanol-containing media (methanol/glucose, methanol/glycerol).

The MOX yields thus found are indicated in Table D. From this it appears that, under optimal conditions, the catalase-negative mutant displays a MOX expression, expressed as units per mg cellular protein, of 49% with respect to the wild-type strain cultured on methanol.

With respect to the wild-type strain cultured on methanol/glucose, the mutant displayed 52-62% expression under optimal conditions.

Example II - Isolation of MOX

Hansenula polymorpha ATCC 46059 was grown on the medium indicated in Table A at a dilution rate of 0.095 hour$^{-1}$. The molar ratio of methanol to glucose was 1.13:1.

Ultrasonic treatment of the cells followed by centrifugation for 15 minutes at 12,000 g yielded an air-stable, cell-free extract.

The MOX activity in the cell-free lysate could be precipitated with a 65% saturated ammonium sulphate solution. The precipitate thus obtained was stable at room temperature. No catalase activity could be detected in the precipitate.

The precipitate obtained with ammonium sulphate can be applied for the uses described in the introductory part of the specification.

Example III - Use of dried cells

Hansenula polymorpha ATCC 46059 was grown on the medium mentioned in Table A at a dilution rate of 0.095 hour$^{-1}$ in a 2.5-litre fermenter. The molar ratio of methanol to glucose was 1.13:1.

The specific activity of MOX, isolated with the aid of the ammonium sulphate method described in Example II, was 3.3 MOX units/mg protein. The (whole) intact cells in suspension displayed a MOX activity of 0.58 MOX units per mg biomass (based on the weight of the dry material). The cells were dried for two days at 15°C under reduced pressure in a freeze-drying apparatus. The activity after renewed suspension of the dried sample in 0.05 M potassium phosphate buffer having a pH of 7.5 and ultrasonic treatment under aerobic conditions was 1.4 MOX units per mg biomass (dry weight). After drying, the cells were stable at room temperature for at least one month. The dried cells contained no catalase activity.

## Table A – Composition of growth media

|  | $g \ l^{-1}$ |
|---|---|
| Glucose | 5 |
| Methanol | 0 – 2.2 |
| $NH_4Cl$ | 7.63 |
| $KH_2PO_4$ | 2.81 |
| $MgSO_4 \cdot 7H_2O$ | 0.59 |
| $CaCl_2 \cdot 2H_2O$ | 0.055 |
| $FeSO_4 \cdot 7H_2O$ | 0.0375 |
| $MnSO_4 \cdot H_2O$ | 0.014 |
| $ZnSO_4 \cdot 7H_2O$ | 0.022 |
| $CuSO_4 \cdot 5H_2O$ | 0.004 |
| $CoCl_2 \cdot 6H_2O$ | 0.0045 |
| $Na_2MoO_4 \cdot 2H_2O$ | 0.0026 |
| $H_3BO_3$ | 0.004 |
| KJ | 0.0026 |
| EDTA | 0.45 |
| Biotine | 0.000075 |
| Thiamine HCl | 0.00625 |
| Meso–inositol | 0.06 |
| Pyridoxine | 0.0015 |
| D–pantothenic acid | 0.03 |

Table B - Growth yield and MOX production of H. polymorpha ATCC 46059 cultured on methanol/ glucose mixtures at a dilution rate of 0.095 hour$^{-1}$

| Molar ratio methanol/glucose | Biomass dry weight g/l | MOX yield Units/mg biomass | Activity Units/mg protein |
|---|---|---|---|
| 0 | 2.55 | 0.12 | 0.28 |
| 0* | 1.50 | 0.16 | 0.4 |
| 0.07 | 2.60 | 0.2 | 0.48 |
| 0.14 | 2.70 | 0.23 | 0.55 |
| 0.2 | 2.76 | 0.32 | 0.76 |
| 0.2 | 2.59 | 0.29 | 0.7 |
| 0.41 | 2.50 | 0.47 | 1.4 |
| 0.81 | 2.59 | 0.73 | 1.74 |
| 1.13 | 2.58 | 1.1 | 2.6 |
| 1.13 | 2.58 | 1.4 | 3.3 |
| 1.8 | 2.53 | 0.81 | 2.5 |
| 1.8 | 2.73 | 0.9 | 2.6 |
| 2.4 | 2.58 | 0.82 | 2.0 |

* vitamin concentration 75% of value in Table A.

Table C — Concentrations of methanol, formaldehyde and and glucose at equilibrium situations

| Ratio methanol : glucose | Residual concentrations | | |
|---|---|---|---|
| | Methanol g/l | Formaldehyde mg/l | Glucose g/l |
| 0.0 | 0 | 0 | 0 |
| 0.014 | 0 | 0 | 0 |
| 0.07 | 0 | 0 | 0 |
| 0.14 | 0 | 0 | 0 |
| 0.2 | 0 | 0 | 0 |
| 0.41 | 0 | 0.28 | 0 |
| 0.8 | 0 | 0.61 | 0 |
| 1.13 | 0 | 0.43 | 0 |
| 1.8 | 0 | 1.1 | 0 |
| 2.4 | 0.17 | 0.47 | 0 |

Table D — Comparison of MOX yields in catalase-free (ATCC 46059, derived from strain CBS 4732) and wild-type H. polymorpha (strain CBS 4732)

| Strain | Substrate concen- tration | Molar ratio | | Yield MOX | |
|---|---|---|---|---|---|
| | | | | Units/mg protein | Units/mg biomass |
| CBS 4732 | 1 % | methanol/glucose | 1.4 | 5.3 | 2.3 |
| CBS 4732 | 0.5% | methanol/glucose | 5.6 | 6.3 | 3.1 |
| CBS 4732 | 5 % | methanol (5% W/W) | | 6.7 | 2.9 |
| CBS 4732 | 5 % | methanol/glycerol | 0.96 | 4.0 | 1.7 |
| ATCC 46059 | 0.6% | methanol/glucose optimal | 1.13 | 3.3 | 1.4 |

**Claims**

1. Process for the preparation of an oxidase or an oxidase-containing composition by aerobic fermentation of a yeast under conditions in which the yeast produces oxidase, and, if desired, isolation from the yeast cells of the oxidase produced, wherein a catalase-negative mutant of a yeast is allowed to grow in a nutritive medium suitable for the yeast in the presence of:
   (a) so-called inducing substrate that induces expression of the oxidase gene and may also be substrate for the oxidase and
   (b) another source of carbon that is suitable for the yeast species chosen,
the molar ratio of inducing substrate to other source of carbon being such that the yeast formed and the oxidase formed do not suffer any harmful effects from the oxidation of the inducing substrate.

2. Process according to claim 1, in which a catalase-negative mutant of a yeast of the genus Hansenula, preferably of the species Hansenula polymorpha, particularly of Hansenula polymorpha 55/11, ATCC 46059, or of the genus Pichia, preferably of the species Pichia pastoris is used.

3. Process according to one or more of the preceding claims, in which, as oxidase, a methanol oxidase is produced and preferably that methanol is used as  inducing substrate.

4. Process according to claim 3, in which a methanol oxidase is produced that has the same amino acid sequence as the known methanol oxidase of Hansenula polymorpha CBS 4732, or a modification of this sequence, obtained via enzyme engineering.

5. Process according to one or more of the preceding claims, in which the yeast is cultured in a nutritive medium that contains another source of carbon, chosen form the group consisting of sugars and other carbon sources used in microbiology.

6. Process according to claim 5, in which methanol and glucose are used as sources of carbon in a molar ratio of methanol: glucose of (0.025-3):1, preferably (1-1.8):1.

7. Process according to one or more of the preceding claims, in which yeast is grown in a continuous fermentation.

8. Process according to one or more of the preceding claims, in which a yeast is used that is modified with the aid of recombinant DNA techniques or its progeny, in which the genetic information for the oxidase stands under control of a strong promoter, preferably a methanol oxidase (MOX) promoter, particularly that of the MOX of Hansenula polymorpha CBS 4732.

9. Process according to one or more of the preceding claims, in which the catalase-negative oxidase-containing yeast is, after finishing the fermentation, subjected to a drying treatment, whereby the oxidas enzyme is not inactivated, preferably a freeze-drying treatment.

10. Process according to one or more of claims 1-8, in which the catalase-negative oxidase-containing yeast is, after finishing the fermentation, subjected to a treatment whereby the cells are made permeable or are inactivated, but the oxidase is not inactivated.


**Patentansprüche**

1. Verfahren zur Herstellung einer Oxidase oder einer Oxidase enthaltenden Zusammensetzung durch aerobe Fermentation einer Hefe unter Bedingungen, bei welchen die Hefe Oxidase produziert, und, nach Wunsch, Isolierung der hergestellten Oxidase aus den Hefezellen, wobei man eine katalasenegative Mutante einer Hefe in einem für die Hefe geeigneten Nährmedium in Anwesenheit
   (a) eines sogenannten induzierenden Substrats, das die Expression des Oxidasegens induziert und auch ein Substrat für die Oxidase sein kann, und
   (b) einer anderen Kohlenstoffquelle, die für die gewählte Hefespezies geeignet ist,
wachsen läßt, wobei das Molverhältnis von induzierendem Substrat zur anderen Kohlenstoffquelle so beschaffen ist, daß die gebildete Hefe und die gebildete Oxidase keine schädigenden Wirkungen aus der Oxidation des induzierenden Substrates erfahren.

2. Verfahren nach Anspruch 1, bei welchem eine katalasenegative Mutante eine Hefe der Art Hansenula, vorzugsweise der Spezies Hansenula polymorpha, insbesondere Hansenula polymorpha 55/11, ATCC 46059 oder der Art Pichia, vorzugsweise der Spezies Pichia pastoris, verwendet wird.

3. Verfahren nach Anspruch 1 oder 2, bei welchem als Oxidase eine Methanoloxidase gebildet und vorzugsweise Methanol als induzierendes Substrat verwendet wird.

4. Verfahren nach Anspruch 3, bei welchem eine Methanoloxidase gebildet wird, die die gleiche Aminosäuresequenz wie die bekannte Mathanoloxidase von Hansenula polymorpha CBS 4732 oder eine Modifikation dieser Sequenz, erhalten durch Enzymmanipulation, aufweist.

5. Verfahren nach einem oder mehreren der vorhergehenden Ansprüche, bei welchem die Hefe in einem Nährmedium gezüchtet wird, das eine andere Kohlenstoffquelle, ausgewählt aus der aus Zuckern und anderen in der Mikrobiologie verwendeten Kohlenstoffquellen bestehenden Gruppe enthält.

6. Verfahren nach Anspruch 5, bei welchem Methanol und Glukose als Kohlenstoffquelle in einem molaren Verhältnis von Methanol zu Glukose von (0.025-3):1, vorzugsweise (1-1,8):1, verwendet werden.

7. Verfahren nach einem oder mehreren der vorhergehenden Ansprüche, bei welchem Hefe in einer kontinuierlichen Fermentation vermehrt wird.

8. Verfahren nach einem oder mehreren der vorhergehenden Ansprüche, bei welchem eine Hefe verwendet wird, die mit Hilfe von rekombinanten DNA-Verfahren modifiziert ist, oder deren Nachkommen, bei welcher sich die genetische Information für die Oxidase unter der Regelung eines starken Promotors, vorzugsweise Methanoloxidase (MOX) - Promotors, insbesondere des der MOX von Hansenula polymorpha CBS 4732, befindet.

9. Verfahren nach einem oder mehreren der vorhergehenden Ansprüche, bei welchem die katalasenegative Oxidase enthaltende Hefe nach der Beendigung der Fermentation einer Trockenbehandlung, durch welche das Oxidaseenzym nicht inaktiviert wird, vorzugsweise einer Gefriertrocknungsbehandlung, unterworfen wird.

10. Verfahren nach einem oder mehreren der Ansprüche 1 bis 8, bei welchem die katalasenegative Oxidase enthaltende Hefe nach der Beendigung der Fermentation einer Behandlung unterworfen wird, durch welche die Zellen permeabel gemacht oder inaktiviert werden, die Oxidase jedoch nicht inaktiviert wird.

## Revendications

1. Procédé de préparation d'une oxidase ou d'une composition contenant de l'oxydase par fermentation aérobie d'une levure dans des conditions dans lesquelles la levure produit de l'oxidase et, éventuellement, l'isolement à partir des cellules de levure de l'oxidase produite, dans lequel un mutant catalase-négatif d'une levure est autorisé à croître dans un milieur nutritif approprié pour la levure en présence de:

(a) ce qu'on appelle un substrat inducteur qui induit l'expression du gène oxidase et peut également constituer un substrat pour l'oxidase, et

.(b) une autre source de carbone qui convient pour le type de levure choisi, le rapport molaire du substrat inducteur à l'autre source de carbone étant tel que la levure formée et l'oxidase formée ne subissent aucun effet fâcheux de l'oxydation du substrat inducteur.

2. Procédé selon la revendication 1, dans lequel on utilise un mutant catalase-négatif d'une levure du genre Hansenula, de préférence du type Hansenula polymorpha et, surtout, Hansenula polymorpha 55/11 ATCC 46059 ou du genre Pichia, de préférence du type Pichia pastoris.

3. procédé selon une ou plusieurs des revendications précédentes dans lequel, à titre d'oxidase, on produit une méthanol-oxidase et on utilise de préférence du méthanol comme substrat inducteur.

4. Procédé selon la revendication 3, dans lequel on produit une méthanol oxidase comportant la même séquence d'amino-acides que la méthanol oxidase connue de Hansenula polymorpha CBS 4732 ou une variante de cette séquence qu'on obtient par manipulation génétique de l'ADN qui code l'enzyme.

5. Procédé selon une ou plusieurs des revendications précédentes, dans lequel on cultive la levure dans un milieu nutritif contenant une autre source de carbone qui peut être un sucre ou une autre source de carbone qu'on utilise en microbiologie.

6. Procédé selon la revendication 5, dans lequel on utilise le méthanol et le glucose comme sources de carbone dans un rapport molaire méthanol:glucose de (0,025-3)/1, de préférence (1-1,8)/1.

7. Procédé selon une ou plusieurs des revendications précédentes, dans lequel on fait pousser la levure par fermentation continue.

8. Procédé selon une ou plusieurs des revendications précédentes dans lequel on utilise une levure modifiée à l'aide des techniques ADN recombinant ou sa lignée, dans lequel l'information génétique pour l'oxidase demeure sous contrôle d'un promoteur fort, de préférence un promoteur de méthanol oxidase (MOX) et, surtout, MOX de Hansenula polymorpha CBS 4732.

9. Procédé selon l'une ou plusieurs des revendications précédentes, dans lequel la levure contenant de l'oxidase catalase-négative est soumise, après achèvement de la fermentation, à un traitement de séchage de sorte que l'enzyme oxidase n'est pas inactivée, de préférence un traitement de lyophilisation.

10. Procédé selon une ou plusieurs des revendications 1 à 8, dans lequel la levure contenant de l'oxidase catalase-négative est soumise, après achèvement de la fermentation, à un traitement par lequel les cellules sont rendues perméables ou sont inactivées, mais l'oxidase n'est pas inactivée.

# Fig.1.

H.POLYMORPHA GROWN ON METHANOL/GLUCOSE
MIXTURES STRAIN ATCC 46059 CATALASE NEGATIVE